(19) Europäisches Patentamt
European Patent Office
Office européen des brevets

(11) Veröffentlichungsnummer: **0 022 075**
**B1**

(12) # EUROPÄISCHE PATENTSCHRIFT

(45) Veröffentlichungstag der Patentschrift: **16.03.83**

(51) Int. Cl.³: **C 07 C 161/00**

(21) Anmeldenummer: **80810197.6**

(22) Anmeldetag: **12.06.80**

(54) **Verfahren zur Herstellung von Bis-(0-(1-Alkylthioäthylimino)-N-methylcarbaminsäure)-N,N'-sulfiden.**

(30) Priorität: **18.06.79 CH 5670/79**

(43) Veröffentlichungstag der Anmeldung: **07.01.81 Patentblatt 81/1**

(45) Bekanntmachung des Hinweises auf die Patenterteilung: **16.03.83 Patentblatt 83/11**

(84) Benannte Vertragsstaaten: **AT BE CH DE FR IT LI LU NL SE**

(56) Entgegenhaltungen:
**CH-A-573 907**
**DE-A-2 530 439**

(73) Patentinhaber: **CIBA-GEIGY AG, Patentabteilung Postfach, CH-4002 Basel (CH)**

(72) Erfinder: **Ledouble, Jean-Pierre, Rue des Landes 10, F-68300 Rosenau (FR)**
Erfinder: **Müller, Klaus, Dr., Steinrebenstrasse 76, CH-4153 Reinach (CH)**

EP 0 022 075 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Herstellung von Bis-[O-(1-Alkylthioäthylimino)-N-methylcarbaminsäure]-N,N'-sulfiden

Die vorliegende Erfindung betrifft ein Verfahren zur Herstellung von Bis-[O-(1-Alkylthioäthylimino)-N-methylcarbaminsäure]-N,N'-sulfiden der Formel

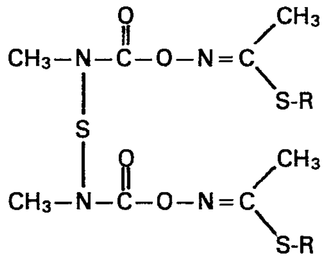

(I)

in welcher R eine geradkettige oder verzweigte Alkylgruppe mi 1 bis 5 Kohlenstoffatomen bedeutet, durch Umsetzung eines O-(1-Alkylthioäthylimino)-N-methylcarbamats der Formel

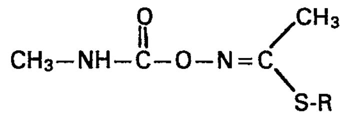

(II)

in welcher R die oben angegebene Bedeutung hat, in Gegenwart einer Base mit Schwefeldichlorid bzw. Dischwefeldichlorid.

Die Bis-[O-(1-Alkylthioäthylimino)-N-methylcarbaminsäure]-N,N'-sulfide der Formel I besitzen insektizide Wirkung und eignen sich insbesondere zur Bekämpfung von Schädlingen von Baumwollpflanzen und Schmeissfliegen (Lucilia sericata).

Es ist bereits aus der Deutschen Offenlegungsschrift 2 530 439 bekannt, die Bis-O-(1-Alkylthioäthylimino)-N-methylcarbaminsäure]-N,N'-sulfide der Formel I durch Umsetzung eines O-(1-Alkylthioäthylimino)-N-methylcarbamats der Formel II in einem inerten Lösungsmittel und in Gegenwart einer Base bei Temperaturen von −10 bis 100°C mit Schwefeldichlorid bzw. Dischwefeldichlorid herzustellen. Dabei werden als inerte Lösungsmittel Äther, aliphatische und aromatische Kohlenwasserstoffe und Ketone und als Basen insbesondere tertiäre Amine, wie Trialkylamine, Pyridine und N,N-Dialkylaniline genannt. Diese Methode ist insofern nachteilig, als die Reaktion bei Verwendung der genannten Lösungsmittel uneinheitlich verläuft, was zu einer Verminderung der Ausbeute und der Produkte-Qualität führt. Ausserdem werden lange Reaktionszeiten benötigt.

Es wurde nun gefunden, dass man die vorgenannten Nachteile vermeiden kann, wenn man die Umsetzung des O-(1-Alkylthioäthylimino)-N-methylcarbamats der Formel II mit Schwefeldichlorid bzw. Dischwefeldichlorid in Gegenwart von 0,01 bis 0,5 Mol pro Mol O-(1-Alkylthioäthylimino)-N-methylcarbamat der Formel II einer Verbindung aus der Gruppe N,N-Dialkylformamid mit je 1 bis 4 Kohlenstoffatomen in den Alkylgruppen, N-Methylpyrrolidon, Dimethylsulfoxid, Phosphorsäure-tris-N,N-dimethylamid und Tetramethylharnstoff durchführt.

Besonders gute Resultate werden erreicht, wenn man die Umsetzung des O-(1-Alkylthioäthylimino)-N-methylcarbamats der Formel II mit Schwefeldichlorid bzw. Dischwefeldichlorid in Gegenwart von N,N-Dimethylformamid durchführt.

Gemäss einer bevorzugten Ausführungsform des erfindungsgemässen Verfahrens wird die Umsetzung eines O-(1-Alkylthioäthylimino)-N-methycarbamats der Formel II mit Schwefeldichlorid bzw. Dischwefeldichlorid in Gegenwart von 0,05 bis 0,2 Mol pro Mol O-(1-Alkylthioäthylimino)-N-methylcarbamat der Formel II einer Verbindung aus der Gruppe N,N-Dialkylformamide mit je 1 bis 4 Kohlenstoffatomen in den Alkylgruppen, N-Methylpyrrolidon, Dimethylsulfoxid, Phosphorsäure-tris-N,N-dimethylamid und Tetramethyharnstoff durchgeführt.

Inerte Lösungsmittel, in denen das erfindungsgemässe Verfahren durchgeführt werden kann, sind aliphatische und aromatische Kohlenwasserstoffe wie Hexan, Cyclohexan, Benzol, p-Xylol und Mesitylen, halogenierte Kohlenwasserstoffe, wie Methylenchlorid, Chloroform, Kohlenstofftetrachlorid, Tetrachloräthylen und 1,1,2-Trifluor-1,2,2-trichloräthan, und Äther, wie Diäthyläther, Tetrahydrofuran und Dioxan. Bevorzugte Lösungsmittel sind 1,1,2-Trifluor-1,2,2-trichloräthan und p-Xylol. Die vorgenannten Lösungsmittel werden vorteilhaft in Mengen von 500 bis 1000 ml, vorzugsweise 500 bis 700 ml pro Mol O-(1-Alkylthioäthylimino)-N-methylcarbamat der Formel II eingesetzt.

Als Basen kommen tertiäre Amine, wie Pyridin, Alkylpyridine, wie Picolin und Lutidin und in p-Stellung durch Halogen, Alkyl und Alkoxy substituierte N,N-Dialkylaniline in Betracht. Vorzugsweise wird Pyridin als Base verwendet. Die vorgenannten Basen können in stöchiometrischer Menge oder in einem Überschuss von bis zu 100% bezogen auf die stöchiometrische Menge eingesetzt werden. Vorzugsweise werden die vorgenannten Basen in einem Überschuss von 50% bezogen auf die stöchiometrische Menge eingesetzt.

Das erfindungsgemässe Verfahren wird bei Temperaturen von -10°C bis +50°C, vorzugsweise 0 bis +30°C durchgeführt. Die Umsetzung nimmt in der Regel weniger als 5 Stunden in Anspruch und ist in den meisten Fällen in 1 bis 2 Stunden beendet.

Das erfindungsgemässe Verfahren wird vorteilhaft in der Weise durchgeführt, dass man ein Gemisch aus Lösungsmittel und Schwefeldichlorid bzw. Dischwefeldichlorid vorlegt, unter Kühlung zunächst die Base, vorzugsweise Pyridin, dann das O-(1-Alkylthioäthylimino)-N-methylcarbamat der Formel II und schliesslich eine Verbindung aus der Gruppe N,N-Dialkylformamide mit je 1 bis 4 Kohlenstoffatomen in den Alkylgruppen, N-Methylpyrrolidon, Dimethylsulfoxid, Phosphorsäure-tris-N,N-dimethylamid und Tetramethylharnstoff, vorzugsweise N,N-Dimethylformamid zugibt und das erhaltene Gemisch 1 bis 2 Stunden bei einer Temperatur von 0 bis 30°C hält.

Die als Ausgangsmaterialien zu verwendenden O-(1-Alkylthioäthylimino)-N-methylcarbamate der Formel II sind aus der britischen Patentschrift

1 138 347 bekannt. Sie können beispielsweise durch Umsetzung von Acetaldehyd mit Hydroxylamin zu Acetaldoxim, weitere Umsetzung des durch Chlorierung von Acetaldoxim erhaltenen Acethydroxamsäurechlorids mit Alkalialkylmercaptid zum entsprechenden 1-Alkylthioacetaldoxim und dessen weitere Umsetzung mit Methylisocyanat hergestellt werden.

Mit dem erfindungsgemässen Verfahren wird es möglich, die Bis-[O-(1-Alkylthioäthylimino)-N-methylcarbaminsäure]-N,N'-sulfide der Formel I unter weitgehender Vermeidung der Bildung unerwünschter Nebenprodukte in Ausbeute zwischen 80 und 90% der Theorie herzustellen. Die Endprodukte werden in einer Reinheit von 95% erhalten und können ohne weitere Reinigung verwendet werden. Ausserdem wird mit dem erfindungsgemässen Verfahren eine wesentliche Verkürzung der Reaktionszeit auf weniger als 5 Stunden erreicht. Das erfindungsgemässe Verfahren ist daher für die Herstellung der insektiziden Wirkstoffe der Formel I in technischem Massstab sehr gut geeignet.

Das erfindungsgemässe Verfahren wird durch die folgenden Beispiele näher erläutert:

*Beispiel 1*

In eine Mischung von 31,0 g (0,30 Mol) Schwefeldichlorid und 285 ml 1,1,2-Trifluor-1,2,2-trichloräthan (Flugen® 113) werden bei 5 bis 10° unter Kühlung 55 g Pyridin eingetropft. Nach beendigter Zugabe des Pyridins werden zunächst 75 g (0,046 Mol) O-(1-Methylthioäthylimino)-N-methylcarbamat und anschliessend 3,3 ml N,N-Dimethylformamid bei 10°C zugegeben. Dann wird das Reaktionsgemisch 2 Stunden bei 25 bis 30°C gerührt und filtriert. Das Produkt wird mit Wasser verrührt, nochmals filtriert und bei 40°C im Vakuum getrocknet. Es werden 77,6 g Bis-[O-(1-methylthioäthylimino)-N-methylcarbaminsäure]-N,N'-sulfid (95%ig) erhalten, was einer Ausbeute von 90% der Theorie, bezogen auf O-(1-Methylthioäthylimino)-N-methylcarbamat, entspricht.

*Beispiel 2*

Nach der in Beispiel 1 beschriebenen Methode werden 31,0 g (0,30 Mol) Schwefeldichlorid und 75 g (0,46 Mol) O-(1-Methylthioäthylimino)-N-methylcarbamat in Gegenwart von 5,6 ml N,N-Dimethylformamid in 300 ml p-Xylol umgesetzt, wobei das Reaktionsgemisch nach Zugabe des N,N-Dimethylformamids 3 Stunden bei 25 bis 30°C gerührt wird. Man erhält auf diese Weise 72,6 g 96%iges Bis-[O-(Methylthioäthylimino)-N-methylcarbaminsäure]-N,N'-sulfid, was einer Ausbeute von 85% der Theorie, bezogen auf O-(1-Methylthioäthylimino)-N-methylcarbamat entspricht.

**Patentansprüche**

1. Verfahren zur Herstellung von Bis-[O-(1-Alkylthioäthylimino)-N-methylcarbaminsäure]-N,N'-sulfiden der Formel I

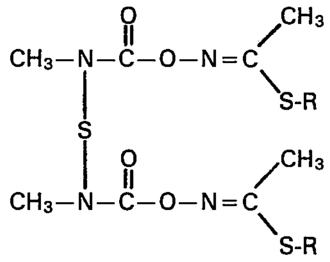

(I)

in welcher R eine geradkettige oder verzweigte Alkylgruppe mit 1 bis 5 Kohlenstoffatomen bedeutet, durch Umsetzung eines O-(1-Alkylthioäthylimino)-N-methylcarbamats der Formel II

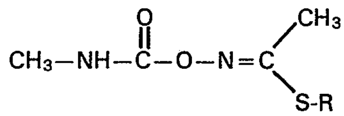

(II)

in welcher R die oben angegebene Bedeutung hat, in Gegenwart einer Base mit Schwefeldichlorid bzw. Dischwefeldichlorid in einem inerten Lösungsmittel, dadurch gekennzeichnet, dass man diese Umsetzung in Gegenwart von 0,01 bis 0,5 Mol pro Mol O-(1-Alkylthioäthylimino)-N-methylcarbamat der Formel II einer Verbindung aus der Gruppe N,N-Dialkylformamid mit je 1 bis 4 Kohlenstoffatomen in den Alkylgruppen, N-Methylpyrrolidon, Dimethylsulfoxid, Phosphorsäure-tris-N,N-dimethylamid und Tetramethylharnstoff durchführt.

2. Verfahren nach Anspruch 1, dadurch gekennzeichnet, dass man die Umsetzung des O-(1-Alkylthioäthylimino)-N-methylcarbamats der Formel II mit Schwefeldichlorid bzw. Dischwefeldichlorid in Gegenwart von N,N-Dimethylformamid durchführt.

3. Verfahren nach Anspruch 1, dadurch gekennzeichnet, dass man die Umsetzung eines O-(1-Alkylthioäthylimino)-N-methylcarbamats der Formel II mit Schwefeldichlorid bzw. Dischwefeldichlorid in Gegenwart von 0,05 bis 0,2 Mol pro Mol O-(1-Alkylthioäthylimino)-N-methylcarbamat der Formel II eine Verbindung aus der Gruppe N,N-Dialkylformamide mit je 1 bis 4 Kohlenstoffatomen in den Alkylgruppen, N-Methylpyrrolidon, Dimethylsulfoxid, Phosphorsäure-tris-N,N-dimethylamid und Tetramethylharnstoff durchführt.

4. Verfahren nach Anspruch 1, dadurch gekennzeichnet, dass man als inerte Lösungsmittel aliphatische und aromatische Kohlenwasserstoffe, halogenierte Kohlenwasserstoffe oder Äther verwendet.

5. Verfahren nach Anspruch 1, dadurch gekennzeichnet, dass man als Lösungsmittel 1,1,2-Trifluor-1,2,2-trichloräthan oder p-Xylol verwendet.

6. Verfahren nach Anspruch 1, dadurch gekennzeichnet, dass man als Base tertiäre Amine verwendet.

7. Verfahren nach Anspruch 1, dadurch gekennzeichnet, dass man als Base Pyridin verwendet.

8. Verfahren nach Anspruch 1, dadurch gekennzeichnet, dass man die Base in einem Überschuss von 50% bezogen auf die stöchiometrische Menge einsetzt.

9. Verfahren nach Anspruch 1, dadurch gekennzeichnet, dass man die Umsetzung eines O-(1-Alkyl-thioäthylimino)-N-methylcarbamats der Formel II mit Schwefeldichlorid bzw. Dischwefeldichlorid bei einer Temperatur von $-10°C$ bis $+50°C$ durchführt.

10. Verfahren nach Anspruch 1, dadurch gekennzeichnet, dass man die Umsetzung eines O-(1-Alkylthioäthylimino)-N-methylcarbamats der Formel II mit Schwefeldichlorid bzw. Dischwefeldichlorid bei einer Temperatur von 0 bis $+30°C$ durchführt.

11. Verfahren nach Anspruch 1, dadurch gekennzeichnet, dass man die Umsetzung eines O-(1-Alkylthioäthylimino)-N-methylcarbamats der Formel II mit Schwefeldichlorid bzw. Dischwefeldichlorid in der Weise durchführt, dass man ein Gemisch aus Lösungsmittel und Schwefeldichlorid bzw. Dischwefeldichlorid vorlegt, unter Kühlung zunächst die Base, dann das O-(1-Alkylthioäthylimino)-N-methylcarbamat der Formel II und schliesslich eine Verbindung aus der Gruppe N,N-Dialkylformamide mit je 1 bis 4 Kohlenstoffatomen in den Alkylgruppen, N-Methylpyrrolidon, Dimethylsulfoxid, Phosphorsäure-tris-N,N-dimethylamid und Tetramethylharnstoff, zugibt und das erhaltene Gemisch 1 bis 2 Stunden bei einer Temperatur von 0 bis 30°C hält.

**Claims**

1. A process for the production of bis-[O-(1-alkylthioethylimino)-N-methylcarbamyl]-N,N'-sulfides of the formula I

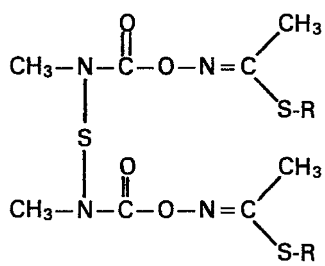

(I)

wherein R is a straight-chain or branched alkyl group of 1 to 5 carbon atoms, by reacting a O-(1-alkylthioethylimino)-N-methylcarbamate of the formula

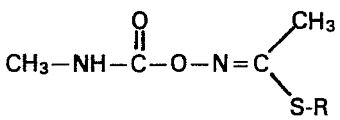

(II)

wherein R is as defined above, in the presence of a base, with sulfur dichloride or sulfur monochloride in an inert solvent, which process comprises carrying out said reaction in the presence of 0.01 to 0.5 mole of a compound selected from the group consisting of N,N-dialkylformamides, containing 1 to 4 carbon atoms in each of the alkyl moieties, N-methylpyrrolidone, dimethylsulfoxide, phosphoric acid tris-N,N-dimethylamide and tetramethyl urea, per mole of O-(1-alkylthioethylimino)-N-methylcarbamate of the formula II.

2. A process according to claim 1, wherein the reaction of the O-(1-alkylthioethylimino)-N-methylcarbamate of the formula II with sulfur dichloride or sulfur monochloride is carried out in the presence of N,N-dimethylformamide.

3. A process according to claim 1, wherein the reaction of an O-(1-alkylthioethylimino)-N-methylcarbamate of the formula II with sulfur dichloride or sulfur monochloride is carried out in the presence of 0.05 to 0.2 mole of a compound selected from the group consisting of N,N-dialkylformamides, containing 1 to 4 carbon atoms in each of the alkyl moieties, N-methylpyrrolidone, dimethylsulfoxide, phosphoric acid tris-N,N-dimethylamide and tetramethyl urea, per mole of O-(1-alkylthioethylimino)-N-methylcarbamate of the formula II.

4. A process according to claim 1, wherein the inert solvent is an aliphatic or aromatic hydrocarbon, a halogenated hydrocarbon or an ether.

5. A process according to claim 1, wherein the solvent is 1,1,2-trifluoro-1,2,2-trichloroethane or p-xylene.

6. A process according to claim 1, wherein the base is a tertiary amine.

7. A process according to claim 1, wherein the base is pyridine.

8. A process according to claim 1, wherein the base is employed in an excess of 50%, based on the stoichiometric amount.

9. A process according to claim 1, wherein the reaction of a O-(1-alkylthioethylimino)-N-methylcarbamate of the formula II with sulfur dichloride or sulfur monochloride is carried out in the temperature range from $-10°C$ to $+50°C$.

10. A process according to claim 1, wherein the reaction of a O-(1-alkylthioethylimino)-N-methylcarbamate of the formula II with sulfur dichloride or sulfur monochloride is carried out in the temperature range from 0°C to $+30°C$.

11. A process according to claim 1, which comprises carrying out the reaction of a O-(1-alkylthioethylimino)-N-methylcarbamate of the formula II with sulfur dichloride or sulfur monochloride by initially adding the base, with cooling, to a ready prepared mixture of solvent and sulfur dichloride or sulfur monochloride, and then adding the O-(1-alkylthioethylimino)-N-methylcarbamate of the formula II and finally a compound selected from the group consisting of N,N-dialkylformamides, containing 1 to 4 carbon atoms in each of the alkyl moieties, N-methylpyrrolidone, dimethylsulfoxide, phosphoric acid tris-N,N-dimethylamide and tetramethyl urea, and keeping the reaction mixture for 1 to 2 hours at a temperature in the range from 0° to 30°C.

**Revendications**

1. Procédé de préparation de N,N'-sulfures de bis-[O-(1-alcoylthioéthylimino)-N-méthyl-acide carbamique] de formule I

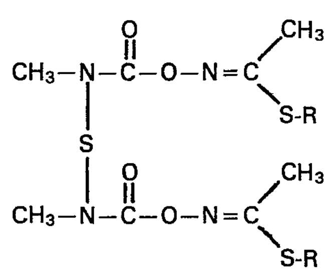

(I)

où R représente un groupe alcoyle à chaîne droite ou ramifiée en $C_1$ à $C_5$, par réaction d'un O-(1-alcoylthioéthylimino)-N-méthylcarbamate de formule II

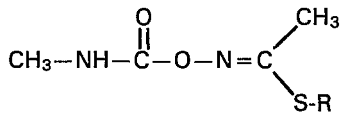

(II)

où R a la signification donnée ci-dessus, en présence d'une base avec du dichlorure de soufre ou du chlorure de soufre dans un solvant inerte, caractérisé en ce qu'on conduit cette réaction en présence de 0,01 à 0,5 mole par mole d'O-(1-alcoylthioéthylimino)-N--méthylcarbamate de formule II d'un composé du groupe des N,N-dialcoylformamides en $C_1$ à $C_4$ dans les groupes alcoyle, de N-méthylpyrrolidone, diméthylsulfoxyde, de tris-N,N-diméthylamide de l'acide phosphorique et de tétraméthylurée.

2. Procédé selon la revendication 1, caractérisé en ce qu'on conduit la réaction du O-(1-alcoylthioéthylimino)-N-méthylcarbamate de formule II avec du chlorure de soufre ou du chlorure de soufre en présence de N,N-diméthylformamide.

3. Procédé selon la revendication 1, caractérisé en ce qu'on conduit la réaction d'un O-(1-alcoylthioéthylimino)-N-méthylcarbamate de formule II avec du dichlorure de soufre ou du chlorure de soufre en présence de 0,05 à 0,2 mole par mole de O-(1--alcoylthioéthylimino)-N-méthyl-carbamate de formule II d'un composés du groupe des N,N-dialcoylformamides en $C_1$ à $C_4$ dans les groupes alcoyle, de N-méthylpyrrolidone, de diméthylsulfoxyde, de tris-N,N-diméthylamide de l'acide phosphorique et de tétraméthylurée.

4. Procédé selon la revendication 1, caractérisé en ce qu'on utilise comme solvants inertes des hydrocarbures aliphatiques et aromatiques, des hydrocarbures halogénés ou des éthers.

5. Procédé selon la revendication 1, caractérisé en ce qu'on utilise comme solvant le 1,1,2-trifluoro-1,2,2-trichloroéthane ou le p-xylène.

6. Procédé selon la revendication 1, caractérisé en ce qu'on utilise comme base des amines tertiaires.

7. Procédé selon la revendication 1, caractérisé en ce qu'on utilise comme base la pyridine.

8. Procédé selon la revendication 1, caractérisé en ce qu'on utilise la base en un excès de 50% par rapport à la quantité stoechiométrique.

9. Procédé selon la revendication 1, caractérisé en ce qu'on conduit la réaction d'un O-(1-alcoylthioéthylimino)-N-méthylcarbamate de formule II avec du dichlorure de soufre ou du chlorure de soufre à une température allant de -10°C à +50°C.

10. Procédé selon la revendication 1, caractérisé en ce qu'on conduit la réaction d'un O-(1-alcoylthioéthylimino)-N-méthylcarbamate de formule II avec du dichlorure de soufre ou du chlorure de soufre à une température allant de 0 à +30°C.

11. Procédé selon la revendication 1, caractérisé en ce qu'on conduit la réaction d'un O-(1-alcoylthioéthylimino)-N-méthylcarbamate de formule II avec du dichlorure de soufre ou du chlorure de soufre de manière à disposer un mélange de solvant et de dichlorure de soufre ou de chlorure de soufre, à commencer par ajouter en refroidissant la base, puis le O-(1-alcoylthioéthylimino)-N-méthylcarbamate de formule II et enfin un composé du groupe des N,N-dialcoylformamides en $C_1$ à $C_4$ dans les groupes alcoyle, de la N-méthylpyrrolidone, du diméthylsulfoxyde, du tris-N,N-diméthylamide de l'acide phosphorique et de la tétraméthylurée, et à maintenir le mélange obtenu pendant 1 à 2 h à une température allant de 0 à 30°C.